Europäisches Patentamt

(19) **European Patent Office**

**Office européen des brevets**

(11) Numéro de publication: **0 205 391**
**B1**

(12) **FASCICULE DE BREVET EUROPEEN**

(45) Date de publication du fascicule du brevet:
**26.10.88**

(51) Int. Cl.⁴: **C 07 C 85/04,** C 07 C 87/62

(21) Numéro de dépôt: **86420128.0**

(22) Date de dépôt: **16.05.86**

(54) **Procédé de préparation de N alcène-2 yl M-trifluorométhylanilines.**

(30) Priorité: **22.05.85 FR 8507694**
**25.10.85 FR 8515858**

(43) Date de publication de la demande:
**17.12.86 Bulletin 86/51**

(45) Mention de la délivrance du brevet:
**26.10.88 Bulletin 88/43**

(84) Etats contractants désignés:
**AT BE CH DE FR GB IT LI LU NL SE**

(56) Documents cités:
**FR - A - 1 140 379**
**FR - A - 2 305 434**
**US - A - 3 005 026**

(73) Titulaire: **RHONE-POULENC CHIMIE, 25, quai Paul Doumer, F-92408 Courbevoie Cédex (FR)**

(72) Inventeur: **Regimbeau, Guy, 33, Rue de Léningrad, F-93300 Bobigny (FR)**
Inventeur: **Augeimann, Gérard, 119, Cours Albert Thomas, F-69003 Lyon (FR)**
Inventeur: **Disdier, Camille, 12, rue Barodet, F-69004 Lyon (FR)**

(74) Mandataire: **Le Pennec, Magali et al, RHONE-POULENC INTERSERVICES Service Brevets Chimie 25, Quai Paul Doumer, F-92408 Courbevoie Cédex (FR)**

ACTORUM AG

## Description

La présente invention concerne un procédé de préparation de N alcène-2 yl métatrifluorométhylanilines. Ces produits sont utilisés comme matières premières pour la fabrication de pyrrolidones halogénées et substituées sur l'atome d'azote.

Il est connu selon le brevet FR 2 305 434 (exemple 7) de préparer les N alcène-2 yl métatrifluorométhylanilines par réaction dans un milieu aprotique polaire en présence d'hydrure de sodium d'un bromure allylique avec une aniline dont un atome d'hydrogène est protégé par condensation avec de l'anhydride acétique. Ce procédé présente l'inconvénient, d'une part, d'utiliser comme matières premières l'hydrure de sodium et un bromure allylique qui sont des produits onéreux et, d'autre part, d'opérer dans un solvant organique; ce procédé se déroule d'autre part en quatre étapes:

— protection de l'amine par acétylation
— substitution de l'hydrogène restant par un métal
— condensation de l'halogénure allylique
— desacétylation.

Un procédé comportant autant d'étapes est souvent difficile à mettre en œuvre du point de vue industriel et est toujours économiquement moins intéressant qu'un procédé en une étape.

Le brevet FR 1 140 379 de la société ETHYLCORPORATION décrit la condensation d'une aniline et d'un dihalogénoalkane en présence d'amine tertiaire avec ou sans solvant. Suivie d'un traitement à la soude pour neutraliser le chlorhydrate et provoquer la cyclisation de l'imine.

Le brevet US 3 005 026 de M. GORDON décrit la condensation de l'aniline avec un halogénure d'alkyle dans l'eau en présence de soude, de carbonate ou de carbonate acide.

Aussi, l'industrie recherche depuis longtemps un procédé économique de préparation de ces composés.

La présente invention a permis de résoudre les problèmes laissés sans solution par l'art antérieur.

Elle a trait à un procédé de préparation de N alcène-2 yl métatrifluorométhylaniline, caractérisé en ce qu'on met en présence en milieu aqueux un halogénure allylique, une métatrifluorométhylaniline, un carbonate, un hydrogénocarbonate ou un hydroxyde alcalin et une quantité catalytique d'une amine tertiaire.

La métatrifluorométhylaniline mise en œuvre dans le procédé selon l'invention peut être constituée de métatrifluorométhylaniline non substituée ou substituée par des éléments n'intervenant pas dans la réaction tels que par exemple des groupements: alkyle, halogènes, alcoxy.

L'halogénure allylique est notamment le bromure ou le chlorure allylique; on préfère cependant utiliser le chlorure allylique car il a un prix nettement inférieur au bromure. On utilise de préférence le chlorure d'allyle.

Le carbonate ou l'hydrogénocarbonate est choisi notamment parmi les carbonates ou hydrogénocarbonate de sodium ou de potassium; on préfère utiliser le carbonate de sodium.

Parmi les hydroxydes alcalins on choisit de préférence la soude ou la potasse.

L'amine teriaire est choisie de façon non limitative parmi la triéthylamine, la tributylamine, la pyridine. Pour une mise en œuvre aisée du procédé selon l'invention, on préfère utiliser une amine tertiaire dont le chlorhydrate est soluble dans l'eau et particulièrement la triéthylamine.

Le procédé objet de la présente invention présente l'avantage par rapport au procédé décrit dans le brevet FR 2 305 434, d'utiliser un milieu de réaction aqueux permettant de travailler en milieu plus concentré et d'éviter les problèmes de recyclage du solvant ce qui permet donc d'abaisser le prix de revient du produit obtenu. D'autres part, ce milieu aqueux permet d'utiliser des bases minérales telles que les carbonates, les hydrogénocarbonates ou les hydroxydes de sodium ou de potassium et ne nécessite l'emploi que de quantités catalytiques d'amines tertiaires. Lorsque l'on opère en milieu organique, les amines sont utilisées généralement en quantité molaire par rapport à l'halogénure allylique ce qui entraîne un recyclage indispensable de la base organique. Ainsi, l'emploi du milieu aqueux et des bases minérales permet des réductions de coût importantes.

La présente concomitante du carbonate ou de l'hydroxyde alcalin et de quantités catalytiques de triéthylamine dans le milieu réactionnel aqueux permet approximativement de doubler la productivité par rapport à la même réaction réalisée en milieu organique en présence de quantités stoechiométriques de triéthylamine par rapport au chlorure allylique.

Selon le procédé de l'invention, on met en œuvre préférentiellement un excès d'aniline par rapport à l'halogénure allylique. Le rapport molaire métatrifluorométhylaniline à l'halogénure allylique est compris de préférence entre 1 et 5 et encore plus préférentiellement, il est d'environ 2.

Le carbonate ou l'hydrogénocarbonate est utilisé selon un rapport: équivalent basique à l'halogénure allylique, compris de préférence entre 1 et 2,5 encore plus préférentiellement, il est d'environ 2.

Le rapport molaire de l'hydroxyde alcalin à l'halogénure allylique est compris entre notamment 1 et 2 et est de préférence d'environ 1.

L'amine tertiaire est utilisée selon un rapport molaire amine tertiaire à l'halogénure allylique, compris de préférence entre 0,01 et 0,20 et encore plus préférentiellement, compris entre 0,05 et 0,10.

La température de la réaction est comprise de préférence entre 50 et 130°C et plus préférentiellement entre 70°C et 100°C.

La réaction est réalisée à pression atmosphérique ou à une pression supérieure. On préfère travailler à pression supérieure quand on travaille à une température au-dessus du point d'ébullition de l'halogénure allylique. La pression sera adaptée à l'économie du procédé.

Les composés issus de la présente invention sont utilisés comme intermédiaire de synthèse dans la fabrication des produits à activité pharmaceutique ou phytosanitaire.

L'invention va être plus complètement décrite à l'aide des exemples suivants qui ne doivent pas être considérés comme limitatifs de l'invention.

*Exemples 1:*
(pas de solvant, base: Na$_2$CO$_3$ aqueux plus quantité catalytique de triéthylamine).

Dans un ballon de 2 l on introduit, sous atmosphère inerte, le carbonate de sodium (318 g: 3 moles), l'eau (0,5 l), la métatrifluorométhylaniline (966 g: 6 moles) et la triéthylamine (20 g: 0,2 mole). Le milieu réactionnel est porté, sous bonne agitation, à t = 50°C. On introduit alors, en 4 heures environ, le chlorure d'allyle (230 g: 3 moles). La température évolue de 50°C à 70°C pendant cette introduction. On porte ensuite la masse réactionnelle à t = 100°C pendant 1 heure, avant de refroidir vers 50°C. Après lavage à l'eau et décantation, la phase organique est rectifiée sous vide (< 5 000 Pa). On sépare ainsi la m-trifluorométhylaniline non transformée (540 g: 3,35 moles) et la N-allyl métatrifluorométhylaniline (367 g: 1,83 moles) ce qui correspond à un rendement de 61% par rapport au chlorure d'allyle et de 70% par rapport à la métatrifluorométhylaniline. La productivité est de 245 g/l de milieu réactionnel.

*Exemple 2:*
(sans solvant, base: Na$_2$CO$_3$ aqueux plus quantité catalytique de triéthylamine).

Dans un ballon de 250 ml on introduit, sous atmosphère inerte, la métatrifluorométhylaniline (97 g: 0,6 mole), le carbonate de sodium (16 g: 0,15 mole), la triéthylamine (2,2 g: 0,02 mole) et 25 ml d'eau. On porte l'ensemble à une température de 70°C et on ajoute en 1 H 30 mn environ, le chlorure d'allyle (23 g: 0,3 mole) et on augmente la température jusqu'à 85°C. On maintient deux heures à 85°C. L'analyse par chromatographie en phase gazeuse du milieu réactionnel indique la présence de N-allyl m. trifluorométhylaniline correspondant à un rendement de 64% environ par rapport au chlorure d'allyle mis en œuvre.

*Exemple 3*
comparatif en milieu organique: (solvant = toluène, base = triéthylamine).

Dans un ballon de 6 l, sous atmosphère inerte, on introduit le toluène (1 830 g) et la m. trifluorométhylaniline (1 127 g: 7 moles). Le milieu réactionnel est agité et porté à t = 110°C. A cette température, le chlorure d'allyle (382,5 g: 5 moles) et la triéthylamine (506 g: 5 moles) sont introduits en parallèle, en 3 heures environ, avec une avance de l'ordre de 10% en chlorure d'allyle. Après la fin de l'introduction, le milieu réactionnel est maintenu 1 heure à t = 115°C puis refroidit à t = 30°C. La masse réactionnelle est lavée à l'eau (4 lavages avec un total 1,3 l d'eau) pour extraction du chlorhydrate de triéthylamine. La phase organique est ensuite rectifiée sous pression atmosphérique, puis sous pression réduite (< 3 000 Pa). On sépare ainsi le toluène, la m. trifluorométhylaniline non transformée (460 g: 2,86 moles) et la N-allyl m. trifluorométhylaniline (588 g: 2,92 moles) ce qui correspond à un rendement de 57% par rapport au chlorure d'allyle et de 70% par rapport à la m. trifluorométhylaniline transformée.

La triéthylamine peut être récupérée par neutralisation de la phase aqueuse avec 200 g de soude (5 moles) à t = 25°C, puis décantation à t B 20°C et distillation. Le taux de récupération atteint 70%. La productivité est de 140 g/l de milieu réactionnel.

*Exemple 4*
comparatif: (solvant: toluène, pas de base additionnelle).

Dans un ballon de 250 ml on introduit, sous azote, le toluène (50 ml), puis la m. trifluorométhylaniline (55,3 g; 0,34 mole). Le mélange est porté à t = 105°C puis additionne, en 2 heures environ, le chlorure d'allyle (10,9 g; 0,14 mole).

On laisse réagir deux heures supplémentaires à t = 105°C.

L'analyse par chromatographie en phase gazeuse du milieu réactionnel montre la présence de faibles quantités de N-allyl m. trifluorométhylaniline correspondant à un rendement de 10% environ par rapport au chlorure d'allyle.

*Exemple 5*
comparatif: (solvant: toluène, base = NaOH aqueuse)

Dans un ballon de 250 ml on introduit, sous azote, le toluène (30 ml) et la métatrifluorométhylaniline (33,8 g; 0,210 mole). Le milieu réactionnel est porté à 75°C et on introduit en parallèle, en 1 H 30 mn environ, le chlorure d'allyle (11,5 g; 0,150 mole) et la soude aqueuse à 20% (6 g; 0,150 mole).

On laisse la réaction se poursuivre 1 heure à t = 75°C. L'analyse par chromatographie en phase gazeuse de la phase organique montre la présence d'une très faible quantité de N-allyl m. trifluorométhylaniline (< 1%).

*Exemple 6*
comparatif: (solvant: toluène, base = K$_2$CO$_3$ solide).

Dans un ballon de 100 ml, on introduit, sous atmosphère inerte, le toluène (15 ml), la m. trifluorométhylaniline (33,8 g: 0,21 mole) et le carbonate de potassium solide (20,7 g: 0,15 mole). On porte le tout à t = 90°C et on ajoute le chlorure d'allyle (11,5 g: 0,15 mole) en 1 H 30 mn environ. On laisse réagir 30 heures à t = 90°C. L'analyse chromatographie phase vapeur du milieu réactionnel indique la présence de N-allyl m. trifluorométhylaniline correspondant à un rendement de 33% par rapport au chlorure d'allyle.

*Exemple 7*
comparatif: (solvant: néant, base = Na$_2$CO$_3$ solide).

Dans un ballon de 250 ml on introduit, sous atmosphère inerte, la métatrifluorométhylaniline (97 g: 0,6 mole) et le carbonate de sodium (16 g: 0,15 mole). On porte le tout à t = 70°C et on ajoute, en 1 H 30 mn environ, le chlorure d'allyle (23 g: 0,3 mole) en augmentant la température à t = 85°C. On maintient encore 2 heures à 85°C.

L'analyse par chromatographie en phase gazeuse indique la présence du N-allyl m. trifluorométhylaniline correspondant à un rendement de 30% environ par rapport au chlorure d'allyle.

*Exemple 8:*
comparatif: (sans solvant, base = Na₂CO₃ aqueux)

Dans un ballon de 250 ml on introduit, sous atmosphère inerte, la m. trofluorométhylaniline (48,3 g: 0,3 mole), l'eau (25 ml) et le carbonate de sodium (8 g: 0,075 mole). On porte le tout à t = 70°C et on ajoute en 1 H 10 le chlorure d'allyle (11,5 g: 0,15 mole) en augmentant la température à 80°C. On maintient encore 2 heures à cette température.

L'analyse par chromatographie en phase gazeuse du milieu réactionnel indique la présence de N-allyl m. trifluorométhylaniline correspondant à un rendement de 30% environ par rapport au chlorure d'allyle.

*Exemple 9:*
(pas de solvant, base = soude aqueuse + quantité catalytique de triéthylamine)

Dans un ballon de 2,1, on introduit, sous atmosphère inerte, la soude aqueuse à 30% (400 g; 3 moles), la métatrifluorométhylaniline (966 g; 6 moles) et la triéthylamine (30 g; 0,3 mole). On porte le milieu réactionnel, sous bonne agitation, à 70°C. On introduit alors le chlorure d'allyle (230 g; 3 moles) en deux heures environ et on maintient ensuite deux heures supplémentaires à 70°C avant de refroidir et de rajouter 320 g d'eau. Après décantation, la phase organique est lavée deux fois à l'eau puis rectifiée sous pression réduite (P < 5 000 Pa).

On sépare ainsi la m. trifluorométhylaniline non transformée (580 g; 3,60 moles) et la N-allyl m- trifluorométhylaniline (355 g; 1,77 moles) ce qui correspond à un rendement de 59% par rapport au chlorure d'allyle et de 74% par rapport à la m. trifluorométhylaniline. La productivité est de 215 g/l de milieu réactionnel.

*Exemple 10*
(pas de solvant, base = soude aqueuse + quantité catalytique de triéthylamine)

Dans un ballon de 150 ml, on introduit, sous atmosphère inerte, la métatrifluorométhylaniline (80,5 g; 0,5 mole), la soude aqueuse à 50% (20 g; 0,25 mole) et la triéthylamine (2,5 g; 0,025 mole). On porte l'ensemble à une température de 70°C et on additionne, en 1 H 30 mn environ, le chlorure d'allyle (19,1 g; 0,25 mole). On maintient ensuite 3 heures à 70°C. L'analyse par chromatographie en phase vapeur du milieu réactionnel montre la présence de N-allyl métatrifluorométhylaniline correspondant à un rendement de 66% environ par rapport au chlorure d'allyle mis en œuvre.

## Revendications

1. Procédé de préparation de N alcène-2 yl m. trifluorométhylaniline caractérisé en ce qu'on met en présence en milieu aqueux un halogénure allylique, une métatrifluorométhylaniline, éventuellement substituée, un carbonate, un hydrogénocarbonate ou un hydroxyde alcalin et une quantité catalytique d'une amine tertiaire.

2. Procédé selon la revendication 1, caractérisé en ce que l'halogénure allylique est le chlorure d'allyle.

3. Procédé selon la revendication 1, caractérisé en ce que le carbonate alcalin est le carbonate de sodium.

4. Procédé selon la revendication 1, caractérisé en ce que l'hydroxyde alcalin est la soude.

5. Procédé selon la revendication 1, caractérisé en ce que l'amine tertiaire est la triéthylamine.

6. Procédé selon la revendication 1, caractérisé en ce que le rapport molaire de la métatrifluorométhylaniline à l'halogénure allylique est compris entre 1 et 5 et de préférence, d'environ 2.

7. Procédé selon la revendication 1, caractérisé en ce que le rapport équivalent basique du carbonate ou de l'hydrogénocarbonate à l'halogénure allylique est compris entre 1 et 2,5 et est de préférence d'environ 2.

8. Procédé selon la revendication 1, caractérisé en ce que le rapport molaire entre l'hydroxyde alcalin et l'halogénure allylique est compris entre 1 et 2 et est de préférence d'environ 1.

9. Procédé selon la revendication 1, caractérisé en ce que le rapport molaire de l'amine tertiaire à l'halogénure allylique est compris entre 0,01 et 0,20 et de préférence entre 0,05 et 0,10.

10. Procédé selon la revendication 1, caractérisé em ce que la température de réaction est comprise entre 50 et 130°C et de préférence entre 70 et 100°C.

## Patentansprüche

1. Verfahren zur Herstellung von N-Alken-2-yl-m-tri-fluormethyl-anilin, dadurch gekennzeichnet, dass in wässrigem Milieu ein Allylhalogenid, eventuell substituiertes Metatrifluormethylanilin, ein Alkalikarbonat, -hydrogenkarbonat oder -hydroxid und eine katalytische Menge eines tertiären Amins miteinander in Kontakt gebracht werden.

2. Verfahren nach Patentanspruch 1, dadurch gekennzeichnet, dass das Allylhalogenid Allylchlorid ist.

3. Verfahren nach Patentanspruch 1, dadurch gekennzeichnet, dass das Alkalikarbonat Natriumkarbonat ist.

4. Verfahren nach Patentanspruch 1, dadurch gekennzeichnet, dass das Alkalihydroxid Ätznatron ist.

5. Verfahren nach Patentanspruch 1, dadurch gekennzeichnet, dass das tertiäre Amin Triethylamin ist.

6. Verfahren nach Patentanspruch 1, dadurch gekennzeichnet, dass das Molverhältnis von Metatrifluormethylanilin zum Allylhalogenid zwischen 1 und 5 liegt und vorzugsweise ungefähr 2 beträgt.

7. Verfahren nach Patentanspruch 1, dadurch gekennzeichnet, dass das Verhältnis der Grammäquivalente Base des Karbonats oder Hydrogenkarbonats zum Allylhalogenid zwischen 1 und 2,5 liegt und vorzugsweise ungefähr 2 beträgt.

8. Verfahren nach Patentanspruch 1, dadurch gekennzeichnet, dass das Molverhältnis des Alkalihydroxides zum Allylhalogenid zwischen 1 und 2 liegt und vorzugsweise ungefähr 1 beträgt.

9. Verfahren nach Patentanspruch 1, dadurch gekennzeichnet, dass das Molverhältnis des tertiären Amins zum Allylhalogenid zwischen 0,01 und 0,20 und vorzugsweise zwischen 0,05 und 0,10 liegt.

10. Verfahren nach Patentanspruch 1, dadurch gekennzeichnet, dass die Reaktionstemperatur zwischen 50 und 130°C und vorzugsweise zwischen 70 und 100°C liegt.

## Claims

1. Process for the preparation of N-2-alkenyl--m-trifluoromethylaniline, characterized in that an allyl halide is brought into contact, in an aqueous medium, with an optionally substituted metatrifluoromethylaniline, an alkali metal carbonate, hydrogen carbonate or hydroxide, and a catalytic quantity of a tertiary amine.

2. Process according to Claim 1, characterized in that the allyl halide is allyl chloride.

3. Process according to Claim 1, characterized in that the alkali metal carbonate is sodium carbonate.

4. Process according to Claim 1, characterized in that the alkali metal hydroxide is sodium hydroxide.

5. Process according to Claim 1, characterized in that the tertiary amine is triethylamine.

6. Process according to Claim 1, characterized in that the molar ratio of meta-trifluoromethylaniline to the allyl halide is between 1 and 5 and preferably approximately 2.

7. Process according to Claim 1, characterized in that the base equivalent ratio of the carbonate or of the hydrogen carbonate to the allyl halide is between 1 and 2.5 and is preferably approximately 2.

8. Process according to Claim 1, characterized in that the molar ratio of the alkali hydroxide to the allyl halide is between 1 and 2 is preferably approximately 1.

9. Process according to Claim 1, characterized in that the molar ratio of the tertiary amine to the allyl halide is between 0.01 and 0.20 and preferably between 0.05 and 0.10.

10. Process according to Claim 1, characterized in that the reaction temperature is between 50 and 130°C and preferably between 70 and 100°C.